# EUROPEAN PATENT SPECIFICATION

(11) **EP 4 069 196 B1**
(45) Date of publication and mention of the grant of the patent: **31.01.2024**
(21) Application number: 20853537.7
(22) Date of filing: 04.12.2020
(51) Int. Cl.: A61K 9/00, A61K 9/08, A61K 35/00, A61K 35/747, A61K 47/02, A61K 47/46

(54) **EYE TOPICAL COMPOSITION CONTAINING METABOLITES OF THE FERMENTATION OF LACTOBACILLUS**
TOPISCHE ZUSAMMENSETZUNG FÜR DAS AUGE MIT METABOLITEN DER FERMENTATION VON LACTOBACILLUS
COMPOSITION OCULAIRE TOPIQUE CONTENANT DES MÉTABOLITES DE LA FERMENTATION DE LACTOBACILLUS

(30) Priority: 05.12.2019 IT 201900023142
(43) Date of publication of application: 12.10.2022
(73) Proprietor: SIFI S.p.A., 95025 Aci Sant'Antonio (CT) (IT)
(72) Inventor: SOLFATO, Elena, 95030 Mascalucia (IT); SUDANO ROCCARO, Andrea, 95024 Acireale (IT); ZAPPULLA, Cristina Maria Concetta, 95127 Catania (IT); SPOTO, Carmela Giovanna, 95022 Acicatena (IT); SPINA, Donato, 95030 Gravina di Catania (IT); PEPE, Veronica, 95030 Mascalucia (IT); SANTONOCITO, Manuela, 95030 Mascalucia (IT); PRICOCO, Angelo, 95030 Mascalucia (IT); VIOLA, Santa, 95010 Sant'Alfio (IT); CURATOLO, Maria Cristina, 95029 Viagrande (IT); MAZZONE, Maria Grazia, 95025 Aci Sant'Antonio (IT); DE PASQUALE, Giuseppe, 95030 Pedara (IT); GIULIANO, Francesco, 95030 Mascalucia (IT)
(74) Representative: Cattaneo, Elisabetta
(86) International application number: PCT/IB2020/061477
(87) International publication number: WO 2021/111372

(56) References cited:
- WO-A1-2018/024833

## Description

### FIELD OF THE INVENTION

The present invention relates to a stable eye topical composition containing metabolites of the fermentation of Lactobacillus.

The invention originates in the ophthalmic filed and that of eye drops.

In particular, the invention relates to a composition for topical use for eye administration containing a postbiotic as the active ingredient and having a specific formulation that keeps the postbiotic in aqueous solution stable.

### BACKGROUND

Probiotics are live micro-organisms that exercise a beneficial effect for the host when administered in biologically active quantities. Postbiotics are subproducts of the fermentation process performed by probiotics.

Postbiotics refer to soluble factors, i.e. metabolic products or subproducts, secreted by live bacteria or released after bacterial lysis, such as enzymes, peptides, teichoic acids, muropeptides, polysaccharides, proteins of the cell surface and organic acids derived from peptidoglycans.

The use of postbiotics in the dietetic and pharmaceutical area is constantly increasing as they are considered to be products with biological activity with a high safety profile.

The use of probiotics in the form of eye drops is also known, for example from Bonni et al.

The use of postbiotics in the ophthalmic sector is difficult because of their low stability in aqueous solution. Patent document WO2018/024833 discloses a composition comprising a fermented product of Lactobacillus casei or paracasei species.

There is therefore a need to have available ophthalmic formulations containing postbiotics that remain stable.

One of the aims of the present invention lies in the fact of providing a composition for topical use for ocular administration containing a postbiotic as a biologically active component.

### SUMMARY

Ophthalmic compositions with high stability containing a specific postbiotic as a biologically active component have now been identified and form the subject matter of the present invention.

Such compositions have a formulation that keeps the postbiotic in the ophthalmic solution stable.

In particular, the compositions of the invention prevent and substantially reduce the protein component of the postbiotic causing instability phenomena such as protein aggregation deriving from the change of the tertiary and quaternary structure of proteins or the denaturation thereof.

According to a first aspect, a composition for eye topical use is provided comprising
a postbiotic which is a fermented product of the Lactobacillus casei or paracasei species,
a buffer system comprising sodium hydrogen phosphate in particular monohydrate, disodium phosphate in particular dodecahydrate,
an isotonising agent comprising magnesium and calcium chloride,
a physiologically acceptable water-based carrier,
said composition having a pH comprised from 6.7 - 6.9 preferably from 6.8 to 6.9.

The compositions according to the invention can further comprise any buffering, isotonising and preservative agents, as described below in detail.

The ophthalmic compositions thus obtained display a surprising ability to act at eye surface level and therefore enable topical therapy to be performed, with high efficacy and a wide safety margin, adapted to prevent or cure diseases in this ocular segment. The new form of therapy can be used in combination with other known therapies for the same disease.

### DESCRIPTION OF THE DRAWINGS

**Figure 1****:** Microbial inhibition percentages obtained from the anti-adhesion assay conducted on two batches at T0 and T6 months under long term stability conditions at 25°C and accelerated at 40°C. The data represent the mean ± SEM of three replicates and the statistical analysis was conducted by means of the one-way ANOVA followed by the Bonferroni test.
**Figure 2****:** (A-B) Percentage of cell viability as a function of the scalar concentrations of the formulations tested according to STRE protocol, under wash/no wash conditions. The data represent the mean ± SEM of three replicates and the statistical analysis was conducted through one sample t-test vs 50% cut-off: **** p < 0.0001 vs cut-off wash condition; ### p< 0.001 and #### p< 0.0001 vs cut-off no wash condition.
**Figure 3****:** (A-E) pH, size, osmolality, pZ and PDI values shown on the graph as a function of study time, under accelerated stability conditions 40 ± 2 ° C / 75 ± 5 RH and long term 25±2°C/60±5% RH.
**Figure 4****:** (A-E) pH, size, osmolality, pZ and PDI values shown on the graph as a function of study time, under accelerated stability conditions 40 ± 2 ° C / 75 ± 5 RH and long term 25±2°C/60±5% RH.
**Figure 5A****:** illustrates a photographic reproduction of the formulation containing postbiotic obtained from the fermentation of Lactobacillus paracasei CNCM I-5220 in water and NaCl according to Example 2, kept for three weeks at 40°C;
**Figure 5B****:** illustrates a photographic reproduction of the formulation containing postbiotic obtained from the fermentation of Lactobacillus paracasei CNCM I-5220 in water and NaCl according to Example 2, kept for one week at 40°C;

### DETAILED DESCRIPTION

The present invention originates from the finding of specific conditions enabling a composition/aqueous solution containing a postbiotic as defined herein to remain stable and the denaturation of its protein components to be prevented or substantially reduced.

These conditions are a) the selection of a pH of the solution in the range from 6.7 to 6.9 preferably from 6.8 to 6.9 using a suitable buffer or buffer system and b) the presence in the solution of bivalent calcium and magnesium salts such as magnesium chloride and calcium chloride.

The Applicant has also observed how the effective regulation of the pH in the desired range is obtained using a buffer of sodium hydrogen phosphate and disodium phosphate.

The subject matter of the present invention is an ophthalmic composition as defined in claim 1.

Further embodiments of the composition are defined in the appended dependent claims 2-9.

The biologically active component of the composition comprises a postbiotic, in particular a product of fermentation from bacterial strains belonging to the Lactobacillus casei or paracasei species, preferably Lactobacillus paracasei.

According to a preferred embodiment, the prebiotic is the strain of Lactobacillus casei CNCMI-1390 filed on 26 July 2017 according to the Budapest Treaty with CNCM (Collection Nationale de Cultures de Microorganismes, Institut Pasteur, Paris, FR) no. I-5220.

According to some embodiments, the composition contains the postbiotic in a quantity from 0.05 to 5%, from 0.1 to 1.9% from 0.2 to 1% weight/volume (g/100ml).

The composition of the invention contains a buffer or buffering system comprising sodium hydrogen phosphate, in particular monohydrate, disodium phosphate in particular dodecahydrate.

Preferably, the ratio between: Sodium hydrogen phosphate monohydrate / disodium phosphate dodecahydrate 16/41=0.39

According to some embodiments, the composition comprises a further buffer comprising sodium citrate.

The composition of the invention comprises an isotonising agent comprising magnesium chloride salts, in particular hexahydrate and calcium chloride, in particular dihydrate.

Preferably, the magnesium chloride is magnesium chloride hexahydrate and the calcium chloride is dihydrate. Preferably, the ratio between: Magnesium chloride hexahydrate / Calcium chloride dihydrate = 10/7=1.42.

According to some embodiments the composition of the invention comprises a further isotonising agent comprising glycerol.

Typically, the composition of the invention contains a physiologically acceptable water-based carrier in which the postbiotic described herein is dispersed. Preferably the water is purified.

The composition of the invention has a pH comprised from 6.7 to 6.9, preferably from 6.8 to 6.9. Therefore, the components of the composition: buffering, isotonising agents are present in the composition of the invention in a quantity such as to obtain a pH from 6.7 to 6.9, preferably from 6.8 to 6.9.

According to one embodiment, the composition of the invention has the following formulation:

| Component | Function | Amount % (w/v) | Range |
|---|---|---|---|
| Postbiotic from *Lactobacillus* casei CNCM n. I-5220 | Bio-surfactant/ Active ingredient | 0.28% | |
| Sodium hydrogen phosphate monohydrate | Buffer | 0.1600 | 0.0350-0.1600 |
| Disodium phosphate dodecahydrate | Buffer | 0.4100 | 0.0890-0.4100 |
| Magnesium chloride hexahydrate | Isotonising agent | 0.0120 | 0.0060-0.0120 |
| Calcium chloride dihydrate | Isotonising agent | 0.0084 | 0.0042-0.0084 |
| Purified water | Solvent | q.s. to 100 mL | - |

According to a preferred form, the composition has the formulation of Example 3 The compositions of the invention are stable, as defined herein.

Furthermore, in the composition, the combination of sodium hydrogen phosphate buffer, preferably monohydrate, and disodium phosphate, preferably dodecahydrate, in quantities such as to provide a pH from 6.7 to 6.9, with an isotonising agent based on magnesium chloride and calcium chloride, make the composition stable.

In the present document, the term "stable" means that the composition has a mean particle dispersion (size) ≤ 200 nm, in particular with reference to the postbiotic, at 40°C and 25°C at six months. Therefore, under the test conditions, no significant variations occur, i.e. increase in size above 200 nm at 25°C or 40°C, attributable to the formation of aggregates of material that originates from the postbiotic.

Within the scope of the present invention, the term postbiotic means a fermented or fermentation product of the species Lactobacillus casei or Lactobacillus paracasei or mixtures thereof and in particular of a fermented product of Lactobacillus paracasei CNCM I-5220.

It has been observed that when the composition according to any one of the previous embodiments has a barrier effect on the epithelial cells, because of the presence of a mixture of biosurfactants, glycoproteins and organic acids and peptides present in the postbiotic, these components of the postbiotic can prevent the binding of pathogenic bacteria on the eye mucosa. Consequently, the composition performs a preventive action or treatment for eye infections.

Furthermore, the composition has immunomodulating activity and can be applied in the prevention and/or treatment of eye allergies and/or inflammations.

The compositions can be in the form of a single dose or multiple dose.

In particular, the buffering component is present in the composition in a concentration such as to obtain/maintain a pH comprised between 5 and 8, compatible with the eye tissues and with the active ingredients carried.

Preferably, the composition is isotonic with the lachrymal liquid and has a value comprised from 270-310 mOsm/Kg.

Preferably, the composition of the invention is an eyewash or an ophthalmic gel.

The compositions according to the invention formulated in multiple doses can also contain antimicrobial preservatives such as, for example: parabens, quaternary ammonium salts, polyhexamethylene biguanide (PHMB) and others possibly comprised among those that can be used in compositions for ophthalmic use. The solvent used in the compositions is preferably water or an aqueous solution of one or more components compatible with topical ophthalmic use.

For administration purposes, the aforesaid compositions can be presented as eye drops or in the form of liquid or gel.

Further subject matter of the invention is the topical ophthalmic use of the compositions as defined above in the preparation of a medication for the treatment or prevention of eye inflammation and/or allergies.

The composition of the invention can be used in the treatment of eye allergies, vernal keratoconjunctivitis and keratoconjunctivitis sicca and blepharitis.

The composition of the invention can also be applied in the prevention of the adhesion of a foreign body or a micro-organism to the surface of the eye, as experimentally demonstrated in the following Example 1.

The following examples further illustrate the invention without constituting any limitation thereof.

### EXAMPLE 1

In the present experimental study, the formulation of Example 3 was used, containing a postbiotic derived from the fermentation process from Lactobacillus paracasei CNCM I-5220, referred to as EyeLac^{®} below. Different assays have been developed for evaluating the anti-adhesive effect, safety and stability of two batches of EyeLac^{®} (R&D1907, R&D1908) composition.

### Methods:

The anti-adhesive property of the formulations R&D1907 and R&D1908 with respect to the carrier (VHC) was evaluated against the strain of *Staphylococcus aureus* (S.a.) ATCC 43300 using the anti-adhesion assay. In particular, 200 µl of each formulation were added to each well and the plate was incubated at 4°C for 18h. After such period, the test item was removed and replaced with the same volume as the bacterial suspension of S.a. ATCC 43300 at the density of 3×10⁸ ufc/ml and the plate incubated for 4h at 4°C, to promote the adhesion of the microrganism. After 4 h, two washes were performed in phosphate-buffered saline (PBS) to remove any bacterial suspension not adhering to the wells, whereas the micro-organisms that had adhered were fixed by adding 200 µl of 99% methanol per well and incubating the plate for 15 minutes at room temperature (RT). Then, the methanol was removed and the plate incubated at 37°C for 5 minutes in order to enable the evaporation of any residual methanol. Finally, the colouring of the adhered micro-organisms was performed by adding to each well 200 µl of 2% crystal violet for 5 minutes at RT. Afterwards, the excess dye was removed and that bound to the micro-organisms in adhesion was dissolved by adding 200 µl of 33% glacial acetic acid per well. The optical density per well was measured by reading at 595 nm on a spectrophotometer. The anti-adhesion assay was performed on all the batches at 25°C and 40°C both at zero time (T0) and at 6 months (T6). The data represent the mean ± SEM of three replicates and the statistical analysis was performed through one-way ANOVA followed by the Bonferroni test.

The **safety** profile of the formulations was evaluated on the human corneal epithelial cell line HCE by means of a cytotoxicity protocol modified with respect to the standard one that envisages repeated exposure to the test item for short treatment periods (STRE). The cells were treated repeatedly (6X) for five minutes with both formulations R&D1907 and R&D1908 at different concentrations (2,5, 5, 10, 20, 100%). After each treatment the protocol followed two different conditions: **Wash:** After 5 minutes of contact on the HCE, the treatment was removed by washing with PBS. This condition represents what happens *in vivo* when the eyelids are closed, in which part of the eyewash is washed away by the tear film. **No Wash:** After 5 minutes of treatment on the HCE, the eyewash is removed without any washing. This condition represents an "exasperated" situation of the previous (wash) and can be compared to a situation in which there is an alteration of the volume of tear film.

In both the protocols described, between one treatment and the following one, there was a "recovery" period of 90 minutes in complete culture medium at 37°C. After the indicated times had passed, the cell viability was evaluated through MTT assay.

Cell viability values less than 50% indicate cytotoxicity (DB-ALM: protocol no. 17). To evaluate the **stability profile** of the formulation two different temperature conditions were taken into consideration: a long term stability study 25±2°C/60±5% RH and accelerated condition 40±2°C/75±5% RH. During the stability study, the following chemical/physical parameters were evaluated: pH value, osmolality and mean particle distribution (size), zeta potential (pZ) and polydispersity index (PDI) at different time intervals (0, 1, 2, 3 and 6 months).

### Results:

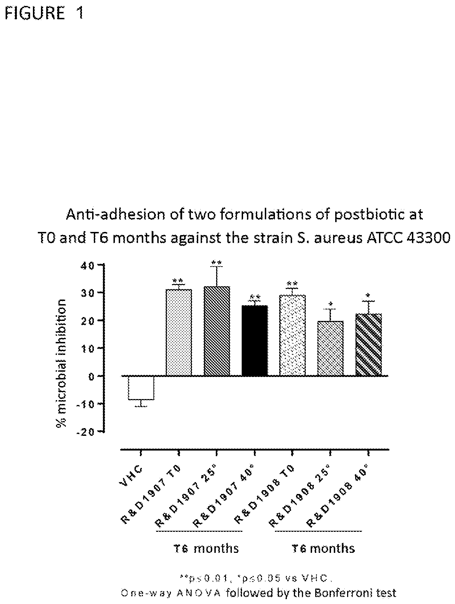

**Anti-adhesion test:** all the batches of the formulation being studied demonstrated anti-adhesive activity towards S.a. ATCC 43300 at both of the temperatures tested, with microbial inhibition values of up to 32.3% with respect to the VHC (Fig. 1). Furthermore, the anti-adhesion effect seems to be kept unaltered up to 6 months. In fact, no statistically significant differences were detected between times T0 and T6 months of either formulation, or between the formulations themselves (R&D1907, R&D1908).

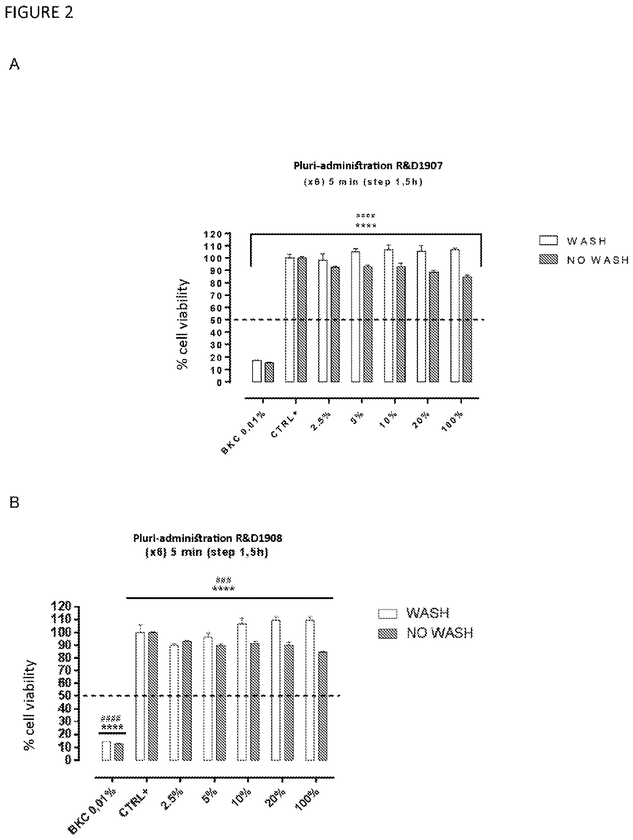

**Safety evaluation:** The results related to the cell viability assay performed on the HCE have demonstrated that the batches R&D1907 and R&D1908, following the treatments performed according to the STRE protocol, are not cytotoxic at any of the tested concentrations either in the wash condition or in the no wash condition (Fig. 2 A-B).

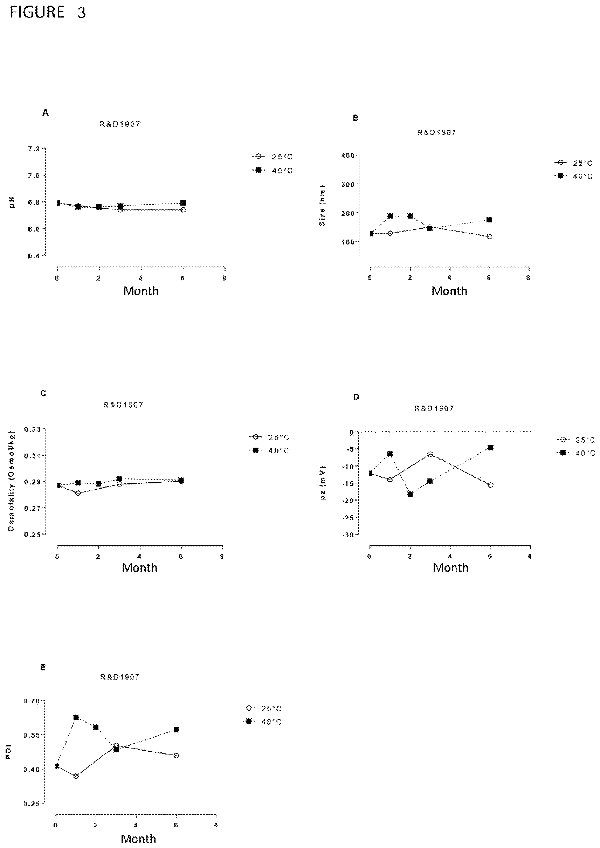

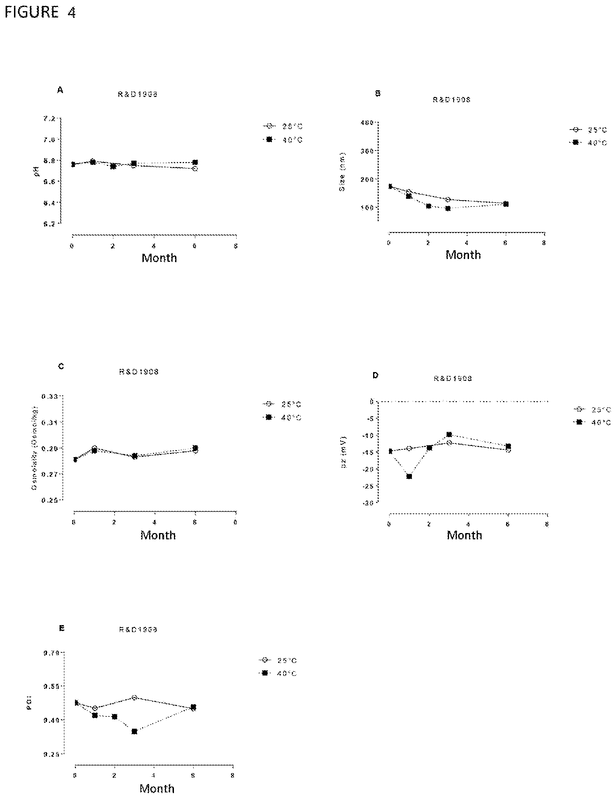

**Stability studies:** The stability study data for the two batches of formulation are provided on the graph as a function of the study times, respectively in Fig.3 (A-E) and Fig.4 (A-E). No significant difference in the chemical-physical parameters (pH, size, PDI, pZ and osmolality) was highlighted during the stability study, either in accelerated stability conditions or in long-term stability conditions. The product can be considered stable.

### Conclusions:

The results suggest that the eye topical formulation is safe, stable and characterised by anti-adhesion activity due to the presence of a mixture of biosurfactants, proteins and organic acids. These results show that such formulation may be useful for preventing diseases in which the barrier function of the conjunctival epithelium is compromised, such as eye infections, allergies and keratoconjunctivitis.

### EXAMPLE 2

Tests were performed for the purpose of verifying how the selection of the pH in the interval from 6.7 to 6.9, in particular from 6.8 to 6.9, is very important for the purpose of keeping the composition stable and preventing the precipitation of protein fractions from the biologically active postbiotic component.

The preferred pH of the formulation tested is equal to 6.8 - 6.9 (***Formulation 1*** of Example 3), whereas the pH of the solution containing the postbiotic obtained from the fermentation of Lactobacillus paracasei CNCM I-5220 - hereinafter referred to as postbiotic - in the absence of any buffer is equal to 6.3.

The composition of the invention has a pH that contributes to maintaining the tertiary and quaternary three-dimensional structure of the proteins of the postbiotic so that they are not denatured and form aggregates. The mean particle dispersion (size) data were optimal for the batches R&D 1907 and 1908 as they have a size ≤ 200 nm and during the stability study both at 40°C and at 25°C until six months, no significant variations were highlighted, i.e. increase in size due to the formation of aggregates.

The presence in the formulation of calcium, magnesium salts contributes to stabilization and the glycerol, as well as having an isotonising function, also has a viscosizing/suspending agent function. The presence of divalent calcium and magnesium salts is very important as they can cause the formation of weak interactions with the (-COO-) groups present in the proteins, stabilising the protein structure.

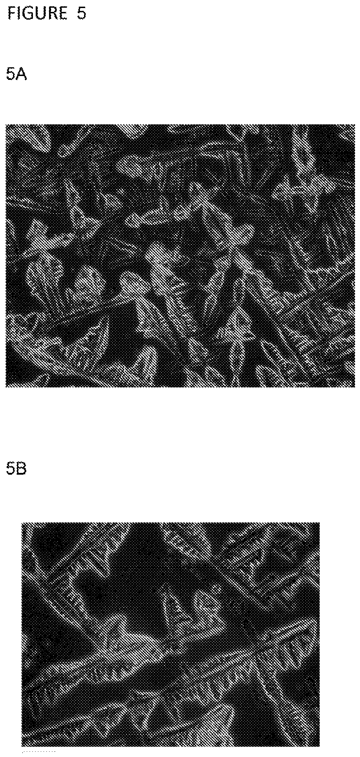

Tests on a formulation in the absence of a buffer system of sodium hydrogen phosphate and disodium phosphate optionally in hydrate form (e.g. monohydrate the first and dodecahydrate the second) but in the presence of isotonising agent have not led to a stable formulation as already after 1 week of study at 40°C the formation of precipitates is observed in the formulation, as illustrated in Figure 5 B.

### Formulation 2 (not according to the claims)

| **Components** | **Function** | **% w/v** |
|---|---|---|
| Postbiotic | Main ingredient | 0,280 |
| NaCl | Isotonising agent | 0,720 |
| Purified water | Solvent | q.s. to 100 |

Other tests were performed on the following buffers with a pH value equal to 5.6-5.8 (formulation 3 and formulation 4), but without obtaining satisfactory results as at lower concentrations of postbiotic (0.1%), the average particle dispersion (size) values obtained are in both cases greater than size > 200 nm. The sample size is high and not very uniform. These factors are an indicator of protein aggregation and the instability of the formulation.

### Formulation 3 (not according to the claims)

| **Components** | **Function** | **%w/v** |
|---|---|---|
| EyeLac^{®} | Main ingredient | 0.100 |
| Na₂HPO4 12H₂O | Buffer | 0.120 |
| Na₂H₂PO4 2H₂O | Buffer | 0.450 |
| Sodium Chloride | Isotonising agent | 0.520 |
| Purified water | Solvent | q.s. to 100 ml |

**Table 1. Formulation 3, Study at T=1 week**

| | | |
|---|---|---|
| **pH** | - | 5.63 |
| **Osmolality** | **Osmol/Kg** | 0.331 |
| **Size** | **nm** | 520 |
| **Pdl** | - | 0.515 |

### Formulation 4 (not according to the claims)

| **Components** | **Function** | **% w/v** |
|---|---|---|
| Postbiotic | Main ingredient | 0.100 |
| Na₂HPO4 12H₂O | Buffer | 0.415 |
| Citric acid | Buffer | 0.880 |
| Purified water | Solvent | q.s. to 100 ml |

**Table 2. Formulation 4, Study at T=1 week**

| | | |
|---|---|---|
| **pH¹** | - | 5.78 |
| **Osmolality¹** | **Osmol/Kg** | 0.239 |
| **Size** | **nm** | 745.60 |
| **Pdl** | - | 0.689 |

### EXAMPLE 3

Formulation of a solution for ophthalmic use

| **Component** | **Function** | **Amount % (w/v)** |
|---|---|---|
| EyeLac | Bio-surfactant/Main ingredient | 0.28% |
| Sodium hydrogen phosphate monohydrate | Buffer | 0.1600 |
| Disodium phosphate dodecahydrate | Buffer | 0.4100 |
| Sodium citrate | Buffer/Isotonising agent | 0.0590 |
| Sodium chloride | Isotonising agent | 0.3500 |
| Potassium chloride | Isotonising agent | 0.1500 |
| Magnesium chloride hexahydrate | Isotonising agent | 0.0120 |
| Calcium chloride dihydrate | Isotonising agent | 0.0084 |
| Glycerol | Isotonising agent | 0.5000 |
| Purified water | Solvent | q.s. to 100 mL |

## Claims

1. A stable eye topical composition comprising
a postbiotic, which is a fermented product of a *Lactobacillus casei* or *Lactobacillus paracasei* species or mixtures thereof,
a liquid carrier, which preferably is water,
a buffer system comprising sodium hydrogen phosphate, preferably monohydrate, and disodium phosphate, preferably dodecahydrate, in amounts to yield a pH from 6.7 to 6.9, preferably from 6.8 to 6.9,
an isotonising agent comprising magnesium chloride salts, in particular hexahydrate and calcium chloride, in particular dihydrate.

2. The composition according to claim 1, wherein the postbiotic is a fermented product of the Lactobacillus casei strain, CNCMI-1390, filed on 26 July 2017 with CNCM (Collection Nationale de Cultures de Microorganismes, Institut Pasteur, Paris, France) no. I-5220.

3. The composition according to claim 1 or 2, comprising a further isotonising agent selected from sodium chloride, potassium chloride and mixture thereof.

4. The composition according to any one of claims 1-3, having the following composition
| Component | Amount % (w/v) |
|---|---|
| Postbiotic from Lactobacillus casei | |
| CNCM no. I-5220 | 0,1-5% |
| Sodium hydrogen phosphate monohydrate | 0,0350-0,1600 |
| Disodium phosphate dodecahydrate | 0,0890-0,4100 |
| Magnesium chloride hexahydrate | 0,0060-0,0120 |
| Calcium chloride dihydrate | 0,0042-0,0084 |
| Purified water | q.s. to 100 mL |

5. The composition according to any one of claims 1-4, further comprising a glycerol based suspending-isotonising agent.

6. The composition according to any one of claims 1-5, comprising a further buffer which is sodium citrate.

7. The composition according to any one of claims 1-6, having the following formulation
| **Component** | **Amount % (w/v)** |
|---|---|
| Postbiotic from Lactobacillus casei | 0.28% |
| CNCM no. I-5220 | |
| Sodium hydrogen phosphate monohydrate | 0.1600 |
| Disodium phosphate dodecahydrate | 0.4100 |
| Sodium citrate | 0.0590 |
| Sodium chloride | 0.3500 |
| Potassium chloride | 0.1500 |
| Magnesium chloride hexahydrate | 0.0120 |
| Calcium chloride dihydrate | 0.0084 |
| Glycerol | 0.5000 |
| Purified water | q.s. to 100 mL |

8. The composition according to any one of claims 1-7, **characterised by** a ratio between: magnesium chloride hexahydrate/calcium chloride dihydrate=10/7=1.42 and/or a ratio sodium hydrogen phosphate monohydrate/disodium phosphate dodecahydrate=16/41=0.39

9. The composition according to any one of claims 1-8, **characterised by** a particle average dispersion (size) ≤ 200 nm at 40°C after six months.

10. The composition according to any one of claims 1-9, for use in preventing or treating microbial eye infections or for treating eye allergies, vernal keratoconjunctivitis, keratoconjunctivitis sicca, blepharitis.

11. The composition according to any one of claims 1-9, for use in preventing an external body or a micro-organism from adhering to the surface of the eye.

## Patentansprüche

1. Stabile, topische Augenzusammensetzung, umfassend
ein Postbiotikum, welches ein fermentiertes Produkt einer *Lactobacillus-casei-* oder *Lactobacillus-paracasei-Art* oder von Mischungen derselben ist, einen flüssigen Träger, der bevorzugt Wasser ist,
ein Puffersystem, das Natriumhydrogenphosphat, bevorzugt Monohydrat, und Dinatriumphosphat, bevorzugt Dodecahydrat, in Mengen umfasst, um einen pH von 6,7 bis 6,9, bevorzugt von 6,8 bis 6,9, zu ergeben,
ein Isotonisierungsmittel, das Magnesiumchloridsalze, insbesondere Hexahydrat, und Calciumchlorid, insbesondere Dihydrat, umfasst.

2. Zusammensetzung nach Anspruch 1, wobei das Postbiotikum ein fermentiertes Produkt des Lactobacillus-casei-Stammes CNCMI-1390, hinterlegt am 26. Juli 2017 bei CNCM (Collection Nationale de Cultures de Microorganismes, Institut Pasteur, Paris, Frankreich), Nr. I-5220, ist.

3. Zusammensetzung nach Anspruch 1 oder 2, umfassend ein weiteres Isotonisierungsmittel, welches ausgewählt ist aus Natriumchlorid, Kaliumchlorid und einer Mischung derselben.

4. Zusammensetzung nach einem der Ansprüche 1 bis 3, welche die folgende Zusammensetzung aufweist:
| Bestandteil | Menge % (w/v) |
|---|---|
| Postbiotikum aus Lactobacillus casei CNCM Nr. I-5220 | 0,1-5% |
| Natriumhydrogenphosphat-Monohydrat | 0,0350-0,1600 |
| Dinatriumphosphat-Dodecahydrat | 0,0890-0,4100 |
| Magnesiumchlorid-Hexahydrat | 0,0060-0,0120 |
| Calciumchlorid-Dihydrat | 0,0042-0,0084 |
| Gereinigtes Wasser | q.s. bis 100 mL |

5. Zusammensetzung nach einem der Ansprüche 1 bis 4, zudem umfassend ein Glycerin-basiertes Suspendierungs-Isotonisierungsmittel.

6. Zusammensetzung nach einem der Ansprüche 1 bis 5, umfassend einen weiteren Puffer, welcher Natriumcitrat ist.

7. Zusammensetzung nach einem der Ansprüche 1 bis 6, welche die folgende Formulierung aufweist:
| Bestandteil | Menge % (w/v) |
|---|---|
| Postbiotikum aus Lactobacillus casei CNCM Nr. I-5220 | 0,28% |
| Natriumhydrogenphosphat-Monohydrat | 0,1600 |
| Dinatriumphosphat-Dodecahydrat | 0,4100 |
| Natriumcitrat | 0,0590 |
| Natriumchlorid | 0,3500 |
| Kaliumchlorid | 0,1500 |
| Magnesiumchlorid-Hexahydrat | 0,0120 |
| Calciumchlorid-Dihydrat | 0,0084 |
| Glycerin | 0,5000 |
| Gereinigtes Wasser | q.s. bis 100 mL |

8. Zusammensetzung nach einem der Ansprüche 1 bis 7, **gekennzeichnet durch** ein Verhältnis zwischen: Magnesiumchlorid-Hexahydrat / Calciumchlorid-Dihydrat = 10/7 = 1,42 und/oder ein Verhältnis Natriumhydrogenphosphat-Monohydrat / Dinatriumphosphat-Dodecahydrat = 16/41 = 0,39.

9. Zusammensetzung nach einem der Ansprüche 1 bis 8, **gekennzeichnet durch** eine mittlere Partikeldispersion (Größe) ≤ 200 nm bei 40°C nach sechs Monaten.

10. Zusammensetzung nach einem der Ansprüche 1 bis 9, zur Verwendung darin, mikrobielle Augeninfektionen zu verhindern oder zu behandeln, oder zum Behandeln von Augenallergien, vernaler Keratokonjunktivitis, Keratoconjunctivitis sicca, Blepharitis.

11. Zusammensetzung nach einem der Ansprüche 1 bis 9, zur Verwendung darin, einen Fremdkörper oder einen Mikroorganismus daran zu hindern, an der Oberfläche des Auges anzuhaften.

## Revendications

1. Une composition oculaire topique stable comprenant
un postbiotique, qui est un produit fermenté d'une espèce *Lactobacillus casei* ou *Lactobacillus paracasei* ou de leurs mélanges,
un support liquide, qui est de préférence de l'eau,
un système tampon comprenant de l'hydrogénophosphate de sodium, de préférence monohydraté, et du phosphate disodique, de préférence dodécahydraté, en quantités pour obtenir un pH de 6,7 à 6,9, de préférence de 6,8 à 6,9,
un agent isotonisant comprenant des sels de chlorure de magnésium, notamment hexahydraté, et de chlorure de calcium, notamment dihydraté.

2. La composition selon la revendication 1, dans laquelle le postbiotique est un produit fermenté de la souche Lactobacillus casei, CNCMI-1390, déposée le 26 juillet 2017 auprès de la CNCM (Collection Nationale de Cultures de Microorganismes, Institut Pasteur, Paris, France) sous le numéro I-5220.

3. La composition selon la revendication 1 ou 2, comprenant un autre agent isotonisant choisi parmi le chlorure de sodium, le chlorure de potassium et leur mélange.

4. La composition selon l'une quelconque des revendications 1-3, ayant la composition suivante
| Composant | Quantité % (poids/volume) |
|---|---|
| Postbiotique de Lactobacillus casei | |
| CNCM n. I-5220 | 0,1 à 5 % |
| Hydrogénophosphate de sodium monohydraté | 0,0350-0,1600 |
| Phosphate disodique dodécahydraté | 0,0890-0,4100 |
| Chlorure de magnésium hexahydraté | 0,0060-0,0120 |
| Chlorure de calcium dihydraté | 0,0042-0,0084 |
| Eau purifiée | q.s. à 100 ml |

5. La composition selon l'une quelconque des revendications 1-4, comprenant en outre un agent suspensif-isotonisant à base de glycérol.

6. La composition selon l'une quelconque des revendications 1-5, comprenant un tampon supplémentaire qui est le citrate de sodium.

7. La composition selon l'une quelconque des revendications 1-6, ayant la formulation suivante
| Composant | Quantité % (poids/volume) |
|---|---|
| Postbiotique de Lactobacillus casei | |
| CNCM n. I-5220 | 0,28% |
| Hydrogénophosphate de sodium monohydraté | 0,1600 |
| Phosphate disodique dodécahydraté | 0,4100 |
| Citrate de sodium | 0,0590 |
| Chlorure de sodium | 0,3500 |
| Chlorure de potassium | 0,1500 |
| Chlorure de magnésium hexahydraté | 0,0120 |
| Chlorure de calcium dihydraté | 0,0084 |
| Glycérol | 0,5000 |
| Eau purifiée | q.s. à 100 ml |

8. La composition selon l'une quelconque des revendications 1-7, **caractérisée par** un rapport entre : chlorure de magnésium hexahydraté/chlorure de calcium dihydraté=10/7=1,42 et/ou un rapport hydrogénophosphate de sodium monohydraté/phosphate disodique dodécahydraté=16/41=0,39.

9. La composition selon l'une quelconque des revendications 1-8, **caractérisée par** une dispersion moyenne des particules (taille) ≤ 200 nm à 40 °C après six mois.

10. La composition selon l'une quelconque des revendications 1-9, pour une utilisation dans la prévention ou le traitement des infections oculaires microbiennes ou dans le traitement des allergies oculaires, de la kératoconjonctivite vernale, de la kératoconjonctivite sèche, de la blépharite.

11. La composition selon l'une quelconque des revendications 1-9, pour une utilisation pour empêcher un corps externe ou un micro-organisme d'adhérer à la surface de l'oeil.
